# EUROPÄISCHE PATENTSCHRIFT

(11) **EP 2 712 919 B1**
(45) Veröffentlichungstag und Bekanntmachung des Hinweises auf die Patenterteilung: **08.11.2017**
(21) Anmeldenummer: 13186208.8
(22) Anmeldetag: 26.09.2013
(51) Int. Cl.: C12N 1/20, C01B 3/00, C02F 3/00

(54) **Wertstoffproduktion mittels halophiler Mikroorganismen**
Production of recycling materials using halophilic microorganisms
Production de substances de valeur au moyen de microorganismes halophiles

(30) Priorität: 27.09.2012 AT 504132012
(43) Veröffentlichungstag der Anmeldung: 02.04.2014
(73) Patentinhaber: Technische Universität Wien, 1040 Wien (AT)
(72) Erfinder: Herwig, Christoph, 1130 Wien (AT); Loranfty, Bettina, 1070 Wien (AT); Martinez Porqueras, Ester, 1090 Wien (AT)
(74) Vertreter: Ellmeyer, Wolfgang

(56) Entgegenhaltungen:
- ES-A6- 2 019 169
- US-A1- 2011 136 196
- DE VRIJE TRUUS ET AL: "Efficient hydrogen production from the lignocellulosic energy crop Miscanthus by the extreme thermophilic bacteria Caldicellulosiruptor saccharolyticus and Thermotoga neapolitana", BIOTECHNOLOGY FOR BIOFUELS, BIOMED CENTRAL LTD, GB, Bd. 2, Nr. 1, 17. Juni 2009 (2009-06-17), Seite 12, XP021060598, ISSN: 1754-6834, DOI: 10.1186/1754-6834-2-12
- KADAR ZSOFIA ET AL: "Hydrogen production from paper sludge hydrolysate.", APPLIED BIOCHEMISTRY AND BIOTECHNOLOGY, Bd. 105-108, April 2003 (2003-04), Seiten 557-566, XP002717233, ISSN: 0273-2289
- KADAR ZSOFIA ET AL: "Yields from glucose, xylose, and paper sludge hydrolysate during hydrogen production by the extreme thermophile Caldicellulosiruptor saccharolyticus", APPLIED BIOCHEMISTRY AND BIOTECHNOLOGY, Bd. 113-116, Nr. Spring, April 2004 (2004-04), Seiten 497-508, XP008103154, ISSN: 0273-2289
- KIVISTO A ET AL: "Hydrogen production from glycerol using halophilic fermentative bacteria", BIORESOURCE TECHNOLOGY, ELSEVIER BV, GB, Bd. 101, Nr. 22, 1. November 2010 (2010-11-01), Seiten 8671-8677, XP027181698, ISSN: 0960-8524 [gefunden am 2010-07-07]
- TSUIHIJI H ET AL: "Cloning and characterization of nif structural and regulatory genes in the purple sulfur bacterium, Halorhodospira halophila", JOURNAL OF BIOSCIENCE AND BIOENGINEERING, ELSEVIER, AMSTERDAM, NL, Bd. 101, Nr. 3, 1. März 2006 (2006-03-01), Seiten 263-270, XP028042326, ISSN: 1389-1723, DOI: 10.1263/JBB.101.263 [gefunden am 2006-03-01]
- ASKER D. ET AL: "Production of canthaxanthin by Haloferax alexandrinus under non-aseptic conditions and a simple, rapid method for its extraction", APPLIED MICROBIOLOGY AND BIOTECHNOLOGY, Bd. 58, Nr. 6, 1. Mai 2002 (2002-05-01), Seiten 743-750, XP055090837, ISSN: 0175-7598, DOI: 10.1007/s00253-002-0967-y
- DATABASE WPI Week 199529 Thomson Scientific, London, GB; AN 1995-220117 XP002717235, & JP H07 132096 A (MICRO ALGE CORP KK) 23. Mai 1995 (1995-05-23)
- DATABASE WPI Week 201275 Thomson Scientific, London, GB; AN 2012-N82414 XP002717236, & CN 102 618 471 A (CHANGZHOU YAHUAN ENVIRONMENTAL PROTECTIO) 1. August 2012 (2012-08-01)
- ED W J VAN NIEL ET AL: "Substrate and product inhibition of hydrogen production by the extreme thermophile, Caldicellulosiruptor saccharolyticus", BIOTECHNOLOGY AND BIOENGINEERING, WILEY & SONS, HOBOKEN, NJ, US, Bd. 81, Nr. 3, 5. Februar 2003 (2003-02-05), Seiten 255-262, XP002661719, ISSN: 0006-3592, DOI: 10.1002/BIT.10463 [gefunden am 2002-12-03]
- ZEIDAN A A ET AL: "A quantitative analysis of hydrogen production efficiency of the extreme thermophile Caldicellulosiruptor owensensis OL<T>", INTERNATIONAL JOURNAL OF HYDROGEN ENERGY, ELSEVIER SCIENCE PUBLISHERS B.V., BARKING, GB, Bd. 35, Nr. 3, 1. Februar 2010 (2010-02-01), Seiten 1128-1137, XP026874337, ISSN: 0360-3199 [gefunden am 2009-12-16]

## Beschreibung

Die vorliegende Erfindung betrifft die Produktion von Wertstoffen durch Kultivierung halophiler Mikroorganismen und Gewinnung der Wertstoffe aus dem Kulturmedium.

### HINTERGRUND DER ERFINDUNG

Alternative Energien haben den Vorteil, dass bei ihrer Verwendung keine schädlichen Treibhausgase (THG) entstehen. Wasserstoff (H₂) ist ein gutes Beispiel, da bei seinem Einsatz in Verbrennungsmotoren Wasser als einziges oxidatives Nebenprodukt entsteht. Verschiedenste Kohlenstoffquellen (Xylose, Glucose, Saccharose, etc.) kommen bei der Produktion von Biowasserstoff zum Einsatz, die durch bestimmte Mikroorganismen mittels verschiedener Stoffwechselwege in Wasserstoff umgewandelt werden. Bekannte Verfahren zur H₂-Produktion sind beispielsweise Biophotolyse, Dunkelfermentation und Photofermentation.

Im Labor werden bei Dunkelfermentationsverfahren strikt anaerobe, extrem thermophile Bakterien, wie z.B. *Caldicellulosiruptor saccharolyticus,* zu diesem Zweck verwendet. *Caldicellulosiruptor saccharolyticus* kann beispielsweise eine Vielzahl von Kohlenhydraten, unter anderem Xylose, zu Kohlendioxid, Wasserstoff, Acetat, Lactat und Ethanol abbauen. Letztere verbleiben in der Fermentationsbrühe.

Diese Herstellungsverfahren wurden weiter optimiert, um die Ausbeute bei der Biowasserstoffherstellung zu erhöhen. So wurden diese Fermentationsbrühen aufgrund ihrer immer noch hohen verfügbaren Restenergie als Ausgangssubstrat für eine zweite Fermentationsstufe verwendet. Dabei kann beispielsweise zuerst eine Dunkelfermentation mit anaeroben Bakterien und danach eine Photofermentation mit photosynthetischen Bakterien durchgeführt werden. Alternativ kann die Dunkelfermentation zur Herstellung von Biowasserstoff als erste Stufe auch mit einem Verfahren zur Herstellung von Methan als zweite Stufe gekoppelt werden.

Halophile Mikroorganismen - kurz "Halophile" - sind Organismen, d.h. im Wesentlichen Bakterien und Archaeen (Archaebakterien), die in Umgebungen mit erhöhter Salzkonzentration leben, wobei unter Salz nicht nur Kochsalz zu verstehen ist, sondern Mineralsalze ganz allgemein. Diese Organismen sind derart an hohe Salzkonzentrationen angepasst, dass sie ihr Wachstum einstellen oder absterben, wenn die Salinität unter eine bestimmte Schwelle sinkt. Je nach Grad der Anpassung unterscheidet man zwischen schwach, moderat und extrem Halophilen. Unter "Haloalkaliphilen" werden jene Halophilen verstanden, die neben dem Salzgehalt ein bestimmtes Mindestmaß an Alkalinität im Medium erfordern.
Dabei weisen "saline" Medien einen Salzgehalt bis zu 3,5 %, ähnlich wie Meerwasser, auf, während Umgebungen, die mehr als 3,5 %, insbesondere mehr als 4 %, Salz enthalten, als "hypersalin" bezeichnet werden.
Für halophile Bakterien sind allgemein die folgenden Anwendungen in der Literatur beschrieben:
- Abwasserbehandlung von hypersalinen Abwasserströmen, die unterschiedliche organische Kohlenstoffquellen enthalten;
- Produktion von Carotinoiden;
- Herstellung von Biokunststoffmaterialien wie PHA, PHB, PHBV;
- Heterologe Überexpression.
Zu den durch Kultierung von Halophilen erhältlichen Wertstoffen zählt beispielsweise Bakterioruberin, ein in der Zellmembran enthaltenes, u.a. als Antioxidans wirkendes Carotinoid, dessen Hauptaufgabe der Schutz des Mikroorganimus gegen UV-Licht ist. Neben dem Erfordernis eines hohen Salzgehalts des Mediums benötigen Halophile zur Vermehrung jedoch eine Reihe von weiteren, definierten Nährstoffen in bestimmten Mindestkonzentrationen, darunter z.B. Kalium, Bromide, Calcium, Magnesium, Ammonium, Phosphat, Sulfat, Carbonat, Mangan und Eisen.
Ziel der Erfindung war vor diesem Hintergrund die Bereitstellung eines Verfahrens zur Gewinnung von Wertstoffen aus Abwasserströmen unter Verwendung von Halophilen. ASKER D. ET AL: "Production of canthaxanthin by Haloferax alexandrinus under non-aseptic conditions and a simple, rapid method for its extraction", APPLIED MICROBIOLOGY AND BIOTECHNOLOGY, Bd. 58, Nr. 6, 1. (2002-05-01), Seiten 743-750 , offenbart die Verwendung von einem Mikroorganismus der Gattung Haloferax alexandrinus für die Herstellung von Carotinoiden unter nicht-sterilen Bedingungen , mit einem Salzzusatz von 20-25 %l NaCl , und Hefeextrakt und Aminosäuren.

### OFFENBARUNG DER ERFINDUNG

Dieses Ziel erreicht die Erfindung durch Bereitstellung der Verwendung eines Abwasserstroms aus der Biowasserstoffproduktion als Kulturmedium zum Züchten eines halophilen Mikroorganismus zur Gewinnung von Wertstoffen, wobei in dem Abwasserstrom zunächst durch Salzzusatz die für das Wachstum des halophilen Mikroorganismus erforderliche Salzkonzentration eingestellt wird, der Mikroorganismus kultiviert wird und in der Folge ein oder mehrere Wertstoffe aus der Fermentationsbrühe gewonnen werden.

Die Erfinder haben nämlich überraschenderweise herausgefunden, dass Abwässer aus der Biowasserstofferzeugung, obwohl diese bereits in einer oder sogar zwei Stufen der Fermentation von Bakterien als Medium gedient haben, sämtliche für das Wachstum von Halophilen erforderliche Komponenten - mit Ausnahme der Salzkonzentration - enthalten. Somit braucht einem solchen Abwasserstom lediglich die erforderliche Menge an Salz zugesetzt zu werden, um ihn als Kulturmedium für Halophile einsetzen zu können. Diese werden dadurch in die Lage versetzt, sich zu vermehren und dabei einen oder mehrere Wertstoffe zu produzieren, die ihrerseits aus dem Fermentationsmedium gewonnen werden können.

Dadurch wird ein Abwasserstrom, der ansonsten verworfen würde - und mitunter vor der Entsorgung auch noch gereinigt werden müsste -, als Ausgangsbasis für die Produktion von Wertstoffen einer wirtschaftlich vorteilhaften Nutzung zugeführt ("waste to value").

Der einzustellende Salzgehalt ist dabei nicht speziell eingeschränkt und hängt vom kultivierten Mikroorganimus ab. Als zuzusetzendes Salz wird vorzugsweise leicht verfügbares Kochsalz, NaCl, als Feststoff oder Lösung verwendet, z.B. eine 1 M NaCl-Lösung. Generell wird ein Gehalt an NaCl im Kulturmedium von 4 bis 30 % (Gew./ Vol.) bevorzugt, noch bevorzugter sind 10 bis 25 %, besonders bevorzugt 20 bis 25 %, um so gleichzeitig die Notwendigkeit des Arbeitens unter sterilen Bedingungen auszuschalten, da eine Kontamination mit anderen Mikroorganismen nahezu ausgeschlossen ist.

Auch wenn der direkt aus der Biowasserstoffproduktion anfallende Abwasserstrom als Kulturmedium für die Fermentation von Halophilen geeignet ist, kann dieser im Bedarfsfall durch gezielte Zusätze ergänzt werden. Darunter fallen beispielsweise pH-Regler, wie z.B. wässrige NaOH oder HCl, zur Einstellung eines für die Kultivierung des jeweiligen Mikroorganismus geeigneten oder optimalen pH-Werts, was speziell im Falle von haloalkaliphilen Organismen von Bedeutung sein kann, aber auch Nährstoffkomponenten, die im Abwasserstrom möglicherweise in unzureichender Menge vorhanden sind, um ein rasches Wachstum des Mikroorganismus zu ermöglichen, sowie Entschäumer und andere, auf dem Gebiet übliche Additive.

Der halophile Mikroorganismus ist nicht speziell eingeschränkt, und es können alle Arten von Bakterien und Archaeen eingesetzt werden, die in der Lage sind, einen oder mehrere Wertstoffe zu produzieren. Beispielsweise kann der Organismus aus halophilen und haloalkaliphilen Mikroorganismen aus der folgenden Gruppe ausgewählt werden: Haladaptatus (Hap.), Halalkalicoccus (Hac.), Haloarcula (Har.), Halobacterium (Hbt.), Halobaculum (Hbl.), Halobiforma (Hbf.), Halococcus (Hcc.), Haloferax (Hfx.), Halogeometricum (Hgm.), Halogranum (Hgn.), Halonotius (Hns.), Halopiger (Hpg.), Haloplanus (Hpn.), Haloquadratum (Hqr.), Halorhabdus (Hrd.), Halorubrum (Hrr.), Halosimplex (Hsx.) Halostagnicola (Hst.), Haloterrigena (Htg.), Halovivax (Hvx.), Natrialba (Nab.), Natrinema (Nnm.), Natronoarchaeum (Nac.), Natronobacterium (Nbt.), Natronococcus (Ncc.), Natronolimnobius (Nln.), Natronomonas (Nmn.), Natronorubrum (Nrr.) und Gemische davon. Besonders gute Ergebnisse wurden bisher mit der Gattung Haloferax (Hfx.) erzielt.

Der zumindest eine zu gewinnende Wertstoff kann beispielsweise ein Biokunststoff, wie oben erwähnt, ein Polysaccharid, ein Carotinoid, wie z.B. Bakterioruberin, oder ein rekombinantes Produkt, wie z.B. ein Protein, sein.

Als Biokunststoffe werden Kunststoffe bezeichnet, die vollständig oder teilweise auf nachwachsenden Rohstoffen basieren, vollständig oder teilweise abbaubar sind oder eine Kombination aus beiden Kriterien darstellen. Wichtige nachwachsende Rohstoffe, die für die Herstellung von Biokunststoffen in Frage kommen, sind unter anderem Stärke bzw. Zucker. Durch Milchsäurebakterien kann Zucker oder Stärke zu Polylactiden (PLA) umgewandelt werden. Auch auf Fettsäuren basierende Biokunststoffe, wie z.B. Polyhydroxyalkanate (PHA), Polyhydroxybutensäure (PHB) oder Poly-(ß-hydroxybutyrat-co-valerat) (PHBV), können bei der Fermentation gewonnen werden.

Zur Gewinnung des zumindest einen Wertstoffs werden die Zellen vorzugsweise vom Fermentationsmedium abgetrennt und gegebenenfalls zerstört, noch bevorzugter durch einfachen Zusatz von Wasser lysiert, d.h. aufgrund des osmotischen Drucks zum Platzen gebracht, und der oder die Wertstoff(e) aus dem Lysat gewonnen, vorzugsweise extrahiert. Die Extraktion des zumindest einen Wertstoffs kann aber auch direkt aus den intakten Zellen erfolgen, z.B. nach moderater Verdünnung mit Wasser, ohne die Zellen platzen zu lassen.

Das erfindungsgemäße Verfahren wird vorzugsweise in einem korrosionsbeständigen Bioreaktor, z.B. aus Edelstahl, noch bevorzugter Glas oder Kunststoff, durchgeführt, beispielsweise in Reaktoren aus Borosilikatglas oder im Labfors PEEK Bioreaktor (aus Polyetheretherketon; erhältlich von INFORS HT, Bottmingen, Schweiz).

### KURZBESCHREIBUNG DER ZEICHNUNGEN

Die vorliegende Erfindung wird nachstehend anhand von Ausführungsbeispielen unter Bezugnahme auf die beiliegenden Zeichnungen näher beschrieben, die Folgendes zeigen:
Fig. 1 die optische Dichte und den Verbrauch von Kohlenstoffquellen, Fig. 2 den pH und den gelösten Sauerstoff und Fig. 3 den Gehalt an Sauerstoff und Kohlendioxid im Abgas während der Kultivierung von Halophilen aus Beispiel 1; und
die Fig. 4 bis 6 dieselben Parameter wie die Fig. 1 bis 3, aber für die Kultivierung aus Beispiel 2.

### BEISPIELE

In den folgenden Beispielen und Vergleichsbeispielen wurde ein Stamm von *Haloferax mediterranei,* hinterlegt unter den Zugriffsnummern DSMZ 1411 bzw. ATCC 33500, einer im Mittelmeer vorkommenden Archaeenart, als Modellorganismus verwendet. Dieser Organismus benötigt zur Vermehrung eine Salzkonzentration von 1,3 bis 4,7 M NaCl mit einem Optimum bei etwa 2,9 M NaCl.

Als Kulturmedium diente der Abwasserstrom einer Biowasserstoffproduktion, die in einer Anlage durchgeführt wurde, die Gegenstand eines Forschungsprojekts am Institut für Verfahrenstechnik, Umwelttechnik und technische Biowissenschaften der Fakultät für Technische Chemie an der Technischen Universität Wien war.

Bei dieser Biowasserstoffproduktion wurde als Medium jenes verwendet, das von A.A. Zeidan und E.W.J. van Niel zur Kultivierung des thermophilen Bakterienstamms *Caldicellulosiruptor owensensis* OL entwickelt wurde; siehe Zeidan, A.A. und E.W.J. Van Niel, "Developing a thermophilic hydrogen-producing co-culture for efficient utilization of mixed sugars", Int. J. Hydrogen Energy 34(10), 4524-4528 (2009), und Zeidan, A.A. und E.W.J. van Niel, "A quantitative analysis of hydrogen production efficiency of the extreme thermophile Caldicellulosiruptor owensensis OL", Int. J. Hydrogen Energy 35(3), 1128-1137 (2010). Es wurde aus den folgenden Komponenten bereitet.

**Tabelle 1 - Medium für die Biowasserstoffproduktion**

| | |
|---|---|
| Xylose | 5 g |
| NH₄Cl | 0,9 g |
| MgCl₂.6H₂O | 0,85 g |
| KH₂PO₄ | 0,75 g |
| K₂HPO₄ | 1,5 g |
| Hefeextrakt | 1 g |
| Cystein-HCl.H₂O | 1 g |

Zu den eingewogenen Komponenten wurde jeweils 1 ml einer Spurenelemente-Lösung und einer Vitamin-Lösung zugesetzt, wonach mit destilliertem Wasser auf 1 Liter aufgefüllt und anschließend ein pH von 6,7 gemessen wurde.

Die Spurenelemente- und die Vitamin-Lösung wurden ihrerseits aus den folgenden Komponenten bereitet:

**Tabelle 2 - Zusammensetzung der Zusatzlösungen**

| Spurenelemente-Lösung | | Vitamin-Lösung | |
|---|---|---|---|
| HCl (25%ig; 7,7 M) | 10 ml | Biotin | 20 mg |
| FeCl₂.4H₂O | 1500 mg | Folsäure | 20 mg |
| ZnCl₂ | 70 mg | Pyridoxin-HCl | 100 mg |
| MnCl₂.4H₂O | 100 mg | Riboflavin | 50 mg |
| H₃BO₃ | 6 mg | Thiamin-HCl | 50 mg |
| CoCl₂.6H₂O | 190 mg | Nicotinamid | 50 mg |
| CoCl₂.2H₂O | 2 mg | Cobalamin | 50 mg |
| NiCl₂.6H₂O | 24 mg | p-Aminobenzoesäure | 50 mg |
| Na₂MoO₄.2H₂O | 36 mg | Liponsäure | 50 mg |
| Na₂WO | 15 mg | Pantothensäure | 50 mg |
| Na₂SeO₃.5H₂O | 15 mg | | |

Die eingewogenen Komponenten wurden jeweils mit dest. Wasser auf 1 Liter aufgefüllt, wovon, wie oben erwähnt, jeweils 1 ml dem Medium für die Biowasserstoffproduktion zugesetzt wurde.

Letztere erfolgte mittels Dunkelfermentation einer anaeroben, extrem thermophilen, cellulolytischen Bakterie, nämlich *Caldicellulosiruptor saccharolyticus* (DSM 8903), die ein Temperaturoptimum von etwa 70°C aufweist. Dabei wurde *C*. *saccharolyticus* zunächst 13 h lang unter Luftausschluss bei 70°C in 100-ml-Fläschen vorkultiviert. Anschließend erfolgte eine zweistufige Biowasserstoffproduktion, zu welchem Zweck 100-150 ml der Vorkultur in einen Bioreaktor überimpft wurden und C. *saccharolyticus* als erste Stufe 8 h lang im Batch-Modus und als zweite Stufe 1 d lang kontinuierlich kultiviert wurde. Die Bedingungen waren in beiden Stufen die folgenden:

**Tabelle 3 - Bedingungen der Biowasserstoffproduktion**

| | |
|---|---|
| Temperatur | 72,5±0,5 °C |
| pH | 6,70±0,03 |
| Rührerdrehzahl | 350 U/min |
| N₂-Strom | 7 l/h |

Mittels Standard-HPLC-Analysen erfolgte eine Identifizierung der bei der Biowasserstoffproduktion erzeugten Metaboliten:
Gerät: Agilent 1100 Series HPLC
Vorsäule: SUPELCOGEL H Guard column (Sigma, 9 µm Teilchengröße, 50 x 4,6 mm)
Säule: SUPELCOGEL C-610H (Sigma, 9 µm Teilchengröße, 300 x 7,8 mm)
Elutionsmittel: 0,1 % H₃P0₄ in destilliertem Wasser, 0,5 ml/min
Detektor: RI

Dabei wurde festgestellt, dass in der Fermentationsbrühe vor allem die folgenden möglichen Kohlenstoffquellen enthalten waren: Xylose als Ausgangssubstrat, sowie als Metaboliten hauptsächlich Acetat, aber auch Lactat und Ethanol.

In der Folge wurde mittels Schüttelkolbenexperimenten untersucht, ob *Haloferax mediterranei* diese Kohlenstoffquellen verwerten könnte, was für alle vier Kohlenwasserstoffe bestätigt wurde, wobei auf Xylose das geringste Wachstum festgestellt wurde.

### Beispiel 1

Nach den somit erfolgreichen Schüttelkolbenexperimenten wurde *Haloferax mediterranei* im Bioreaktor (Labfors PEEK Bioreaktor; INFORS HT, Bottmingen, CH) mit dem Abwasser aus der Biowasserstoffproduktion kultiviert, dem lediglich 200 g/l NaCl zugesetzt wurden.

**Tabelle 4 - Zusätze zum Abwasser**

| | |
|---|---|
| NaCl | 200 g/l |

Die Parameter der Kultivierung waren die folgenden:

**Tabelle 5 - Bedingungen der Halophilen-Fermentation**

| | |
|---|---|
| Temperatur | 38 °C |
| pH | 7,2 |
| Rührerdrehzahl | 400 U/min |
| Luftstrom | 0,4 l/h |

Während der Kultivierung wurden die folgenden Parameter überwacht:
- der Verbrauch der Kohlenstoffquellen in der Fermentationsbrühe durch die halophilen Bakterien mittels HPLC;
- die optische Dichte bei 600 nm, die mit dem Biomassewachstum korreliert;
- pH- und pO₂-Werte
- Abgasdaten: O₂ und CO₂

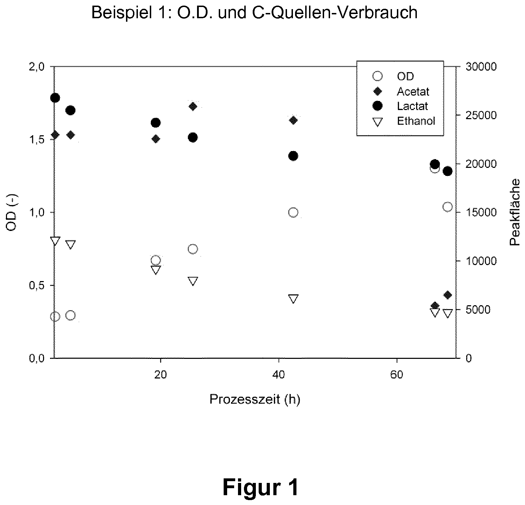

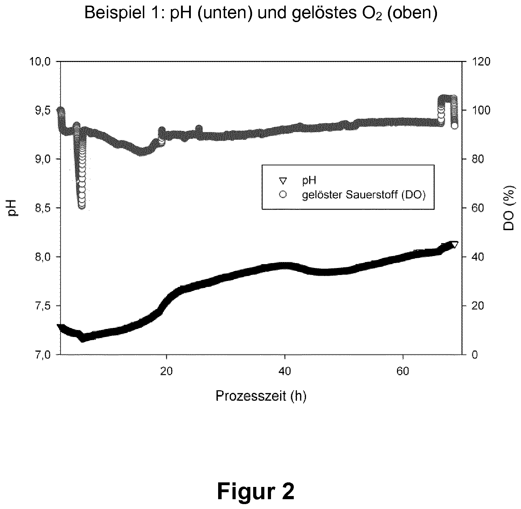

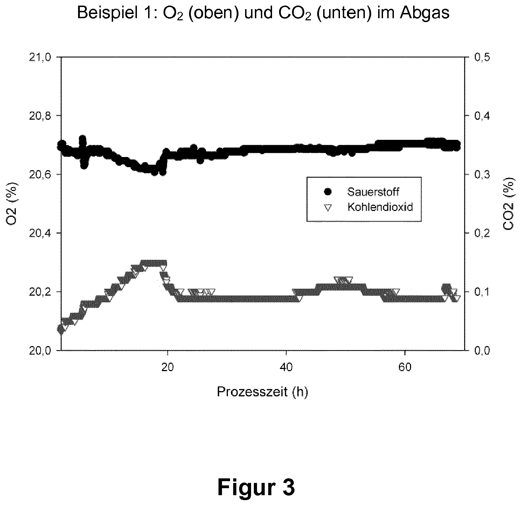

In den Fig. 1 bis 3 sind die Ergebnisse der Parameter-Messungen grafisch dargestellt. Dabei zeigt Fig. 1 die optische Dichte und den Verbrauch der Kohlenstoffquellen Acetat, Lactat und Ethanol, Fig. 2 den pH und den gelösten Sauerstoff (in %) und Fig. 3 den Gehalt an Sauerstoff und Kohlendioxid im Abgas.

Die exponentielle Zunahme der optischen Dichte in Fig. 1 zeigt klar das Wachstum des Halophilen-Stamms, vor allem während der ersten 40 h der Kultivierung, was auch gut mit der Abnahme der Konzentrationen (durch die Peakflächen ausgedrückt) der Kohlenstoffquellen in Fig. 1, der Abnahme des pH und der Zunahme an gelöstem Sauerstoff in Fig. 2 sowie der Zunahme des CO₂ im Abgas in Fig. 3 korreliert. Aus Fig. 1 geht weiters hervor, dass zunächst Ethanol und Lactat und erst später, nach etwa 30-40 h, auch Acetat als Kohlenstoffquellen verbraucht wurden.

Die halophile Archaee *Haloferax mediterranei* war somit sehr gut in der Lage, sich im Abwasser einer Biowasserstoffproduktion zu vermehren, dem lediglich Kochsalz zuzusetzen war.

### Beispiel 2

Zur Untersuchung, ob durch Ergänzung des Mediums eine Steigerung des Wachstums erzielbar ist, wurde in diesem Beispiel dem Abwasser aus der Biowasserstoffproduktion die nachstehenden Komponenten zugesetzt.

**Tabelle 6 - Zusätze zum Abwasser**

| | |
|---|---|
| NaCl | 200 g/l |
| Spurenelemente-Lösung | 1 ml |
| CaCl₂.6H₂O | 1 g/l |
| KCl | 5 g/l |
| NaHCO₃ | 0,2 g/l |
| KBr | 0,5 g/l |

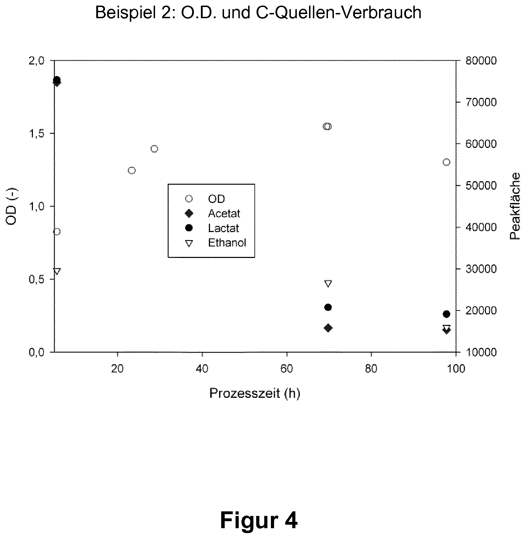

Die sonstigen Bedingungen der Kultivierung waren dieselben wie in Beispiel 1, und in den Fig. 4 bis 6 sind die Ergebnisse der Messungen derselben Parameter wie im vorhergehenden Beispiel dargestellt.

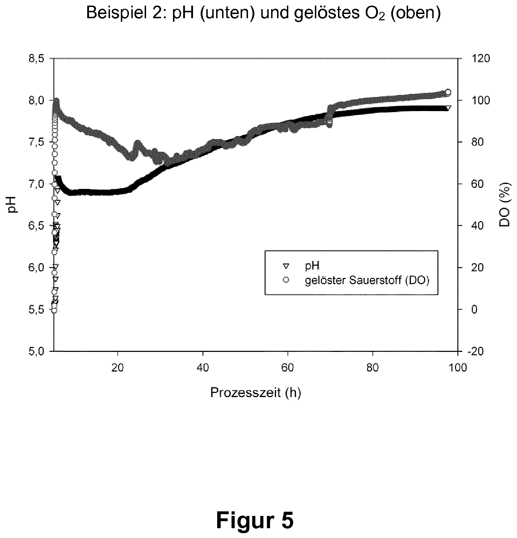

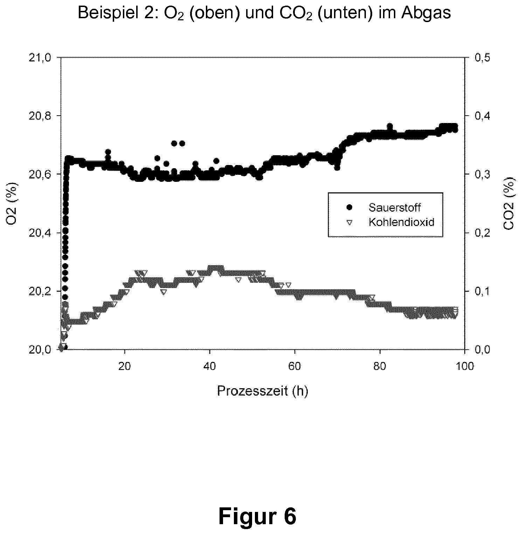

Fig. 4 zeigt dabei eine deutlich höhere optische Dichte als Fig. 1, was ein vermehrtes Wachstum des Organismus anzeigt. Dies korreliert wiederum mit dem Verbrauch der als Kohlenstoffquellen in Fig. 4, der Abnahme des pH und der Zunahme an gelöstem Sauerstoff in Fig. 5 sowie der Zunahme des CO₂ im Abgas in Fig. 6. Interessanterweise wurden im vorliegenden Beispiel jedoch zunächst Acetat und Lactat und erst später, d.h. nach etwa 50-60 h, auch Ethanol als Kohlenstoffquellen verbraucht.

Die Tatsache, dass generell alle Kohlenstoffquellen verbraucht wurden, zeigt jedoch, dass die Kohlenstoffquelle nicht der limitierende Faktor bei der Kultivierung in Beispiel 1 war, sondern eine oder mehrere der sonstigen Komponenten, die in Beispiel 2 ergänzt wurden. Diese Komponente(n) war(en) offenbar im Kulturmedium aus Beispiel 1 in zwar für generelles Wachstum des Organismus ausreichender, aber dennoch limitierender Menge enthalten.

Bei der praktischen Ausführung der vorliegenden Erfindung obliegt es demnach dem Fachmann, den genauen Nährstoffbedarf des jeweils eingesetzten halophilen Organismus, soweit bekannt, entweder nachzulesen oder durch einfache Reihenversuche zu bestimmen und den für die Kultivierung eingesetzten Abwasserstrom aus der Biowasserstoffproduktion gegebenenfalls entsprechend zu ergänzen.

Auf diese Weise können auf Basis solcher Abwässer optimale Wachstumsbedingungen für diverse halophile Mikroorganismen geschaffen werden, wodurch deren Wertstoffproduktion optimiert werden kann, obwohl die vorliegende Erfindung durchaus auch mit einem unveränderten Abwasser als Kulturmedium ausführbar ist, wie Beispiel 1 klar belegt.

### Wertstoffgewinnung

Wie zuvor erwähnt, sind die auf diese Weise gewinnbaren Wertstoffe nicht speziell eingeschränkt und hängen unmittelbar vom verwendeten Halophilen-Stamm und von den diesem zur Verfügung gestellten Substraten ab. Das heißt, der als Kulturmedium dienende Abwasserstrom aus der Biowasserstoffproduktion kann durchaus auch mit geeigneten Nährstoffquellen ergänzt werden, die die Produktion bestimmter Wertstoffe ermöglichen.

Der hierin verwendete Oganismus *Haloferax mediterranei* produziert beispielsweise ein extrazelluläres Heteropolysaccharid in Mengen von bis zu 3 mg/ml, das in einfacher Weise, z.B. durch Fällung mit Ethanol aus dem kalten Kulturüberstand gewonnen werden kann und als Ölbinder und Verdicker nützlich sein kann (siehe Anton et al., Appl. Environ. Microbiol. 54(10), 2381-2386 (1988)).

Andere, von Halophilen produzierte (z.B. intrazellulär vorliegende) Wertstoffe können hingegen leichter zugänglich sein, wenn die Zellen - gegebenenfalls nach vorheriger Isolierung aus der Fermentationsbrühe - zerstört werden. Im Falle von Halophilen ist dies auf besonders einfache Weise möglich, nämlich durch simples Verdünnen der Brühe mit Wasser, was in vielen Fällen schon ein Aufplatzen der Zellen bewirkt. Anschließend können die Wertstoffe auf fachbekannte Weise aus dem Lysat gewonnen werden, z.B. durch Extraktion, Fällung, Destillation oder dergleichen.

Und schließlich ist eine riesige Palette gewünschter Wertstoffe nach genetischer Modifikation der halophilen Bakterien bzw. Archaeen zugänglich, um diese dadurch in die Lage zu versetzen, rekombinante Produkte, darunter vor allem diverse Proteine, wie z.B. Hormone, zu exprimieren.

Die vorliegende Erfindung stellt demnach einen wertvolle Ergänzung des Standes der Technik auf dem Gebiet der Biotechnologie dar.

## Patentansprüche

1. Verwendung eines Abwasserstroms aus einer Wertstoffproduktion als Kulturmedium zum Fermentieren von Mikroorganismen zur Produktion eines oder mehrerer weiterer Wertstoffe, die aus der Fermentationsbrühe gewonnen werden,
**dadurch gekennzeichnet, dass**:
a) ein halophiler Mikroorganismus fermentiert wird,
b) Abwasser aus einer Biowasserstoffproduktion als Kulturmedium eingesetzt wird, wobei
c) das Abwasser aus einer zweistufigen Biowasserstoffproduktion stammt; und
d) in dem Abwasserstrom zunächst durch Salzzusatz die für das Wachstum des halophilen Mikroorganismus erforderliche Salzkonzentration eingestellt wird, bevor dieser kultiviert wird.

2. Verwendung nach Anspruch 1, **dadurch gekennzeichnet, dass** im Kulturmedium ein Salzgehalt von 4 bis 30 % (Gew./Vol.), z.B. 10 bis 25 % oder 20 bis 25 %, eingestellt wird.

3. Verwendung nach Anspruch 1 oder 2, **dadurch gekennzeichnet, dass** dem Kulturmedium zur Einstellung des Salzgehalts Kochsalz oder Kochsalzlösung zugesetzt wird.

4. Verwendung nach einem der Ansprüche 1 bis 3, **dadurch gekennzeichnet, dass** das Kulturmedium durch Zusatze ergänzt wird.

5. Verwendung nach einem der vorangegangenen Ansprüche, **dadurch gekennzeichnet, dass** die Kultivierung des halophilen Mikroorganismus unter nicht-sterilen Bedingungen erfolgt.

6. Verwendung nach einem der vorangegangenen Ansprüche, **dadurch gekennzeichnet, dass** der Organismus aus halophilen und haloalkaliphilen Mikroorganismen aus der folgenden Gruppe ausgewählt wird: Haladaptatus (Hap.), Halalkalicoccus (Hac.), Haloarcula (Har.), Halobacterium (Hbt.), Halobaculum (Hbl.), Halobiforma (Hbf.), Halococcus (Hcc.), Haloferax (Hfx.), Halogeometricum (Hgm.), Halogranum (Hgn.), Halonotius (Hns.), Halopiger (Hpg.), Haloplanus (Hpn.), Haloquadratum (Hqr.), Halorhabdus (Hrd.), Halorubrum (Hrr.), Halosimplex (Hsx.) Halostagnicola (Hst.), Haloterrigena (Htg.), Halovivax (Hvx.), Natrialba (Nab.), Natrinema (Nnm.), Natronoarchaeum (Nac.), Natronobacterium (Nbt.), Natronococcus (Ncc.), Natronolimnobius (Nln.), Natronomonas (Nmn.), Natronorubrum (Nrr.) und Gemische davon.

7. Verwendung nach Anspruch 6, **dadurch gekennzeichnet, dass** ein Mikroorganismus der Gattung Haloferax (Hfx.) eingesetzt wird.

8. Verwendung nach einem der vorangegangenen Ansprüche, **dadurch gekennzeichnet, dass** der zumindest eine zu gewinnende Wertstoff aus Polysacchariden, Biokunststoffen, Carotinoiden und rekombinanten Produkten ausgewählt ist.

9. Verwendung nach einem der vorangegangenen Ansprüche, **dadurch gekennzeichnet, dass** zur Gewinnung des zumindest einen Wertstoffs die Zellen des Mikroorganismus vom Fermentationsmedium abgetrennt und gegebenenfalls zerstört werden.

10. Verwendung nach Anspruch 9, **dadurch gekennzeichnet, dass** die abgetrennten Zellen durch Zusatz von Wasser lysiert werden und der zumindest eine Wertstoff aus dem dabei entstehenden Lysat extrahiert wird.

## Claims

1. A use of a waste water stream from the production of useful substances as a culture medium for fermenting microorganisms in order to produce one or more further useful substances which are obtained from the fermentation broth, **characterized in that:**
a) a halophilic microorganism is fermented,
b) waste water from the production of biohydrogen is used as said culture medium, wherein
c) the waste water originates from a two-step biohydrogen production process and
d) the salt concentration required for the growth of said halophilic microorganism is obtained by adding salt to the waste water stream before the microorganism is cultivated.

2. The use according to claim 1, **characterized in that** the salt concentration of the culture medium is adjusted to 4 to 30 % (w/v), such as to 10 to 25 % or 20 to 25 %.

3. The use according to claim 1 or claim 2, **characterized in that** sodium chloride or saline is added to the culture medium to adjust the salt content.

4. The use according to any one of the claims 1 to 3, **characterized in that** the culture medium is complemented by additives.

5. The use according to any one of the preceding claims, **characterized in that** the halophilic microorganism is cultivated under non-sterile conditions.

6. The use according to any one of the preceding claims, **characterized in that** the organism is selected from halophilic and haloalkaliphilic microorganisms from the following group: *Haladaptatus* (Hap.), *Halalkalicoccus* (Hac.), *Haloarcula* (Har.), *Halobacterium* (Hbt.), *Halobaculum* (Hbl.), *Halobiforma* (Hbf.), *Halococcus* (Hcc.), *Haloferax* (Hfx.), *Halogeometricum* (Hgm.), *Halogranum* (Hgn.), *Halonotius* (Hns.), *Halopiger* (Hpg.), *Haloplanus* (Hpn.), *Haloquadratum* (Hqr.), *Halorhabdus* (Hrd.), *Halorubrum* (Hrr.), *Halosimplex* (Hsx.) *Halostagnicola* (Hst.), *Haloterrigena* (Htg.), *Halovivax* (Hvx.), *Natrialba* (Nab.), *Natrinema* (Nnm.), *Natronoarchaeum* (Nac.), *Natronobacterium* (Nbt.), *Natronococcus* (Ncc.), *Natronolimnobius* (Nln.), *Natronomonas* (Nmn.), *Natronorubrum* (Nrr.), and mixtures thereof.

7. The use according to claim 6, **characterized in that** a microorganism of the *Haloferax* (Hfx.) species is used.

8. The use according to any one of the preceding claims, **characterized in that** said at least one useful substance to be obtained is selected from polysaccharides, bioplastics, carotenoids, and recombinant products.

9. The use according to any one of the preceding claims, **characterized in that** the cells of the microorganism are separated from the fermentation medium, and optionally disrupted, to obtain said at least one useful substance.

10. The use according to claim 9, **characterized in that** said separated cells are lysed by adding water and said at least one useful substance is extracted from the thus obtained lysate.

## Revendications

1. Utilisation d'un flux d'eaux usées provenant d'une production d'un matériau utilisable comme milieu de culture pour la fermentation de microorganismes afin de produire un ou plusieurs matériaux utilisables supplémentaires, qui sont obtenus à partir du bouillon de fermentation,
**caractérisée en ce que**:
a) un microorganisme halophile est fermenté;
b) des eaux usées provenant d'une production de biohydrogène sont employées comme milieu de culture, cependant
c) les eaux usées sont issues d'une production de biohydrogène à deux étapes;
d) la concentration saline nécessaire pour la croissance du microorganisme halophile est d'abord, avant qu'il soit cultivé, ajustée dans le flux d'eaux usées par l'addition de sel.

2. Utilisation selon la revendication 1, **caractérisée en ce que** une salinité de 4 à 30 % (p/vol), p.ex. de 10 à 25 % ou de 20 à 25 %, est ajustée dans le milieu de culture.

3. Utilisation selon les revendications 1 ou 2, **caractérisée en ce que** du chlorure de sodium ou de la solution saline sont ajoutés au milieu de culture afin d'ajuster la salinité.

4. Utilisation selon une quelconque des revendications 1 à 3, **caractérisée** en ce le milieu de culture est complété par des additifs.

5. Utilisation selon une quelconque des revendications précédentes, **caractérisée en ce que** la culture du microorganisme halophile est effectuée dans des conditions non stériles.

6. Utilisation selon une quelconque des revendications précédentes, **caractérisée en ce que** l'organisme est sélectionné parmi des microorganismes halophiles et halo-alcalinophiles du groupe suivant : *Haladaptatus* (Hap.), *Halalkalicoccus* (Hac.), *Haloarcula* (Har.), *Halobacterium* (Hbt.), *Halobaculum* (Hbl.), *Halobiforma* (Hbf.), *Halococcus* (Hcc.), *Haloferax* (Hfx.), *Halogeometricum* (Hgm.), *Halogranum* (Hgn.), *Halonotius* (Hns.), *Halopiger* (Hpg.), *Haloplanus* (Hpn.), *Haloquadratum* (Hqr.), *Halorhabdus* (Hrd.), *Halorubrum* (Hrr.), *Halosimplex* (Hsx.) *Halostagnicola* (Hst.), *Haloterrigena* (Htg.), *Halovivax* (Hvx.), *Natrialba* (Nab.), *Natrinema* (Nnm.), *Natronoarchaeum* (Nac.), *Natronobacterium* (Nbt.), *Natronococcus* (Ncc.), *Natronolimnobius* (Nln.), *Natronomonas* (Nmn.), *Natronorubrum* (Nrr.) ou un mélange de ceux-ci.

7. Utilisation selon la revendication 6, **caractérisée en ce qu'**un microorganisme de l'espèce *Haloferax* (Hfx.) est employé.

8. Utilisation selon une quelconque des revendications précédentes, **caractérisée en ce que** l'au moins un matériau utilisable à obtenir est sélectionné parmi des poly-saccarides, des bioplastiques, des caroténoïdes et des produits recombinants.

9. Utilisation selon une quelconque des revendications précédentes, **caractérisée en ce que**, afin d'obtenir l'au moins un matériau utilisable, les cellules du microorganisme sont séparées du milieu de fermentation et éventuellement détruites.

10. Utilisation selon la revendication 6, **caractérisée en ce que** les cellules séparées sont lysées par l'addition d'eau et **en ce que** l'au moins un matériau utilisable est extrait du lysat ainsi obtenu.
